# EUROPEAN PATENT APPLICATION

(11) **EP 4 779 651 A1**
(43) Date of publication of application: **22.07.2026**
(21) Application number: 24865675.3
(22) Date of filing: 26.07.2024
(51) Int. Cl.: G16H 50/70, G16H 50/20, G16H 10/60, A61B 5/16, G16H 10/20, G16H 50/50, G06N 3/0464

(54) **METHOD FOR PREDICTING DEPRESSION BY MEASURING EMOTIONAL SYMPTOMS AND DEVICE FOR PERFORMING SAME**

(30) Priority: 13.09.2023 KR 20230121721
(71) Applicant: Korea Institute of Science & Technology Information, Daejeon 34141 (KR)
(72) Inventor: YANG, He Young, Seoul 04425 (KR); YUN, Min Young, Seoul 02461 (KR); JEON, Min Jeong, Seoul 07525 (KR)
(74) Representative: GLAWE DELFS MOLL
(86) International application number: PCT/KR2024/010896
(87) International publication number: WO 2025/058232

(57) **Abstract**

According to an aspect of the present disclosure, a method for predicting depression is performed by a computing device, and may comprise: generating a plurality of time-series data by measuring a plurality of emotional symptoms of a user for a specific period; generating emotional network data based on the generated time-series data; generating interconnection network data by calculating a correlation coefficient between the time-series data of the generated emotional network data; and predicting a depression symptom of the user after the specific period by applying a machine learning algorithm to the emotional network data or the interconnection network data.

## Description

### CROSS-REFERENCE TO RELATED APPLICATION

This application is a Continuation of International Application No. PCT/KR2024/010896 filed on July 26, 2024, which claims benefit of priority to Korean Patent Application No. 10-2023-0121721 filed on September 13, 2023, the entire content of which is incorporated herein by reference.

### BACKGROUND

### 1. Technical Field

The present disclosure relates to a method for predicting depression by measuring emotional symptoms and an apparatus for performing the same, and more particularly, to a method for predicting whether depression symptoms will worsen in the future by constructing a network using emotional symptom measurement values for a plurality of days and applying a machine learning algorithm to the constructed network.

### 2. Description of the Related Art

As the number of patients suffering from depression is increasing globally, becoming a significant social issue, diagnosis and early prediction of depression are becoming increasingly important. However, due to the nature of depressed patients, they often do not visit hospitals voluntarily, but clinical diagnosis still relies on standard diagnostic procedures based on questionnaire responses from patients who visit hospitals, and clinically useful and definitive biomarkers have not yet been unknown. Currently, as biomarkers for diagnosing depression, biological biomarkers measured by medical institutions, such as brain images, neurotransmitters, growth factors and hormones, and digital biomarkers measured through digital devices, such as moving distances, call history and sleep states, are being extensively researched.

The biological biomarkers are limited in social utility because they can only be measured upon visiting a medical institution. In case of the digital biomarkers, more comprehensive early prediction of depression may be possible, but it is difficult to expand their application because they require large-scale data and the provision of personal life information. Accordingly, there is a need for a method for diagnosing and early predicting depression with high accuracy without requiring visiting a medical institution or excessive demands for personal information.

### SUMMARY

An object of the present disclosure is to provide a method for predicting depression by using voluntarily measured emotional symptom values.

Another object of the present disclosure is to provide a method for predicting depression by quantifying emotional symptoms from a usage history of a digital device such as a smartphone.

According to an aspect of the present disclosure, a method for predicting depression is performed by a computing device, and may comprise: generating a plurality of time-series data by measuring a plurality of emotional symptoms of a user for a specific period; generating emotional network data based on the generated time-series data; generating interconnection network data by calculating a correlation coefficient between the time-series data of the generated emotional network data; and predicting a depression symptom of the user after the specific period by applying a machine learning algorithm to the emotional network data or the interconnection network data.

In one embodiment, the plurality of emotional symptoms may include a plurality of positive emotional symptoms and a plurality of negative emotional symptoms.

In one embodiment, the generating emotional network data may include: normalizing each of the generated time-series data to a same scale; interpolating a missing value of each of the generated time-series data; and generating matrix data including the plurality of time-series data, for which the normalization and the interpolation have been performed, as the emotional network data.

In one embodiment, the predicting a depression symptom may include: predicting whether the depression symptom will worsen by applying the machine learning algorithm to the interconnection network data; and predicting a change trend of an emotion corresponding to the time-series data by applying the machine learning algorithm to the time-series data of the emotional network data and the interconnection network data.

In one embodiment, the machine learning algorithm may be a Graph Convolutional Neural Network (Graph CNN) algorithm.

In one embodiment, the generating a plurality of time-series data may include generating the plurality of time-series data by receiving a result of measuring the plurality of emotional symptoms of the user from the user for the specific period.

In one embodiment, the generating a plurality of time-series data may include generating the plurality of time-series data by quantifying a result of estimating the plurality of emotional symptoms from the user's usage history of a digital device for the specific period.

In one embodiment, the plurality of emotional symptoms may be estimated by extracting keywords representing emotions from text acquired from the usage history of the digital device.

According to another aspect of the present disclosure, a computing device may comprise: a processor; and a memory storing instructions, wherein the instructions, when executed by the processor, may cause the processor to perform operations of: generating a plurality of time-series data by measuring a plurality of emotional symptoms of a user for a specific period; generating emotional network data based on the generated time-series data; generating interconnection network data by calculating a correlation coefficient between the time-series data of the generated emotional network data; and predicting a depression symptom of the user after the specific period by applying a machine learning algorithm to the emotional network data or the interconnection network data.

In one embodiment, the plurality of emotional symptoms may include a plurality of positive emotional symptoms and a plurality of negative emotional symptoms.

In one embodiment, the generating emotional network data may include: normalizing each of the generated time-series data to a same scale; interpolating a missing value of each of the generated time-series data; and generating matrix data including the plurality of time-series data, for which the normalization and the interpolation have been performed, as the emotional network data.

In one embodiment, the predicting a depression symptom may include: predicting whether the depression symptom will worsen by applying the machine learning algorithm to the interconnection network data; and predicting a change trend of an emotion corresponding to the time-series data by applying the machine learning algorithm to the time-series data of the emotional network data and the interconnection network data.

In one embodiment, the machine learning algorithm may be a Graph Convolutional Neural Network (Graph CNN) algorithm.

In one embodiment, the generating a plurality of time-series data may include generating the plurality of time-series data by receiving a result of measuring the plurality of emotional symptoms of the user from the user for the specific period.

In one embodiment, the generating a plurality of time-series data may include generating the plurality of time-series data by quantifying a result of estimating the plurality of emotional symptoms from the user's usage history of a digital device for the specific period.

In one embodiment, the plurality of emotional symptoms may be estimated by extracting keywords representing emotions from text acquired from the usage history of the digital device.

According to still another aspect of the present disclosure, an apparatus for predicting depression may comprise: a measurement module generating a plurality of time-series data by measuring a plurality of emotional symptoms of a user for a specific period; a data generation module generating emotional network data based on the generated time-series data, and generating interconnection network data by calculating a correlation coefficient between the time-series data of the generated emotional network data; and a prediction module predicting a depression symptom of the user after the specific period by applying a machine learning algorithm to the emotional network data or the interconnection network data.

### BRIEF DESCRIPTION OF THE DRAWINGS

The above and other aspects and features of the presently disclosed technology will become more apparent by describing in detail exemplary embodiments thereof with reference to the attached drawings, in which:
FIG. 1 is a block diagram illustrating an exemplary configuration of an apparatus for predicting depression according to an embodiment of the present disclosure.
FIG. 2 illustrates emotional network data and interconnection network data according to an embodiment of the present disclosure.
FIG. 3 is a flow chart illustrating a method for predicting depression according to an embodiment of the present disclosure.
FIG. 4 is a detailed flow chart illustrating a step of generating emotional network data of FIG. 3.
FIG. 5 is a detailed flow chart illustrating a step of predicting depression symptoms of FIG. 3.
FIG. 6 is a flow chart illustrating a method for predicting depression according to another embodiment of the present disclosure.
FIG. 7 is a block diagram illustrating hardware configuration of a computing device for predicting depression according to an embodiment of the present disclosure.

### DETAILED DESCRIPTION OF THE EMBODIMENTS

Preferred embodiments of the present disclosure will hereinafter be described in detail with reference to the accompanying drawings. The advantages, features, and methods of achieving them of the present disclosure will become clearer with the embodiments described in detail along with the accompanying drawings. However, the present disclosure is not limited to the embodiments described below and can be implemented in various different forms. These embodiments are provided only to make the disclosure complete and fully inform those of ordinary skill in the technical field to which the present disclosure belongs, and the present disclosure is defined only by the scope of the claims.

It is noted that the same reference numerals are used for the same elements across different drawings as far as possible. Furthermore, in describing the present disclosure, detailed descriptions of known configurations or functions will be omitted when they may obscure the essence of the present disclosure.

Unless defined otherwise, all terms used herein (including technical and scientific terms) can have the meaning commonly understood by one of ordinary skill in the art to which the present disclosure belongs. Terms defined in commonly used dictionaries are not interpreted in an ideal or excessive manner unless explicitly defined otherwise. The terms used in the present specification are for the purpose of describing particular embodiments only and are not intended to limit the invention. In this specification, the singular forms include plural forms unless the context clearly indicates otherwise.

Furthermore, in describing the components of the present disclosure, terms such as first, second, A, B, (a), (b), etc., may be used. These terms are intended to distinguish the components from others, and the essence, order, or sequence of such components is not limited by these terms. If a component is stated as being "connected," "coupled," or "linked" to another component, the component can be directly connected or linked to the other component, but it should be understood that there may also exist other components "connected," "coupled," or "linked between them.

The terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

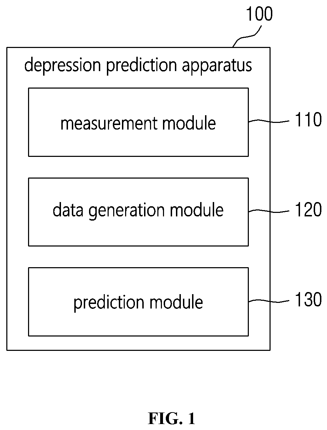

FIG. 1 is a block diagram illustrating an exemplary configuration of an apparatus 100 for predicting depression according to an embodiment of the present disclosure. Hereinafter, the apparatus 100 for predicting depression will be referred to as a depression prediction apparatus 100. The depression prediction apparatus 100 may generate emotional network data based on individual's voluntary emotional symptom measurement values for a plurality of days, and may predict whether depression symptoms will worsen in the future by applying a machine learning algorithm to the generated emotional network data.

Referring to FIG. 1, the depression prediction apparatus 100 may include a measurement module 110, a data generation module 120, and a prediction module 130. It should be noted that components (modules) of the depression prediction apparatus 100, which are shown in FIG. 1, represent functional elements that are functionally classified, and at least one component (module) may be implemented in a form of being integrated with another one in an actual physical environment.

The measurement module 110 may generate time-series data obtained by measuring individual's emotional symptoms for a specific period. To this end, the measurement module 110 may provide a user with a series of interfaces for voluntarily measuring emotional symptoms (i.e., for receiving individual's measurement results). For example, the emotional symptoms may be classified into positive emotional symptoms (e.g., cheerfulness, comfort, joy, etc.) and negative emotional symptoms (e.g., sadness, anger, fear, etc.), and each symptom may be measured as a quantified value (e.g., a Likert scale of 1 to 7 points). Alternatively, the emotional symptoms may be measured depending on valence representing a degree of positivity or negativity of an emotion, and arousal representing a degree of excitement caused by the emotion, based on Russell's circumplex model of emotion. For convenience of description, it is assumed that the measurement module 110 according to an embodiment of the present disclosure measures positive emotional symptoms and negative emotional symptoms, but the present disclosure is not limited thereto, and the measurement module 110 may measure the individual's emotional symptoms through various questionnaires that serve as scales for diagnosing depression.

According to an embodiment, the measurement module 110 may quantify emotional symptoms based on an individual's usage history of a digital device. For example, the measurement module 110 may acquire text, such as the individual's search history, content of documents read on the web, content of posts written on social networking services (SNS), and content of posts "liked" on the SNS, and may estimate and quantify emotional symptoms by extracting keywords related to emotions from the acquired text. The quantified emotional symptoms may similarly be generated as time-series data. The time-series data of the emotional symptom measurement values generated through the measurement module 110 may be provided to the data generation module 120.

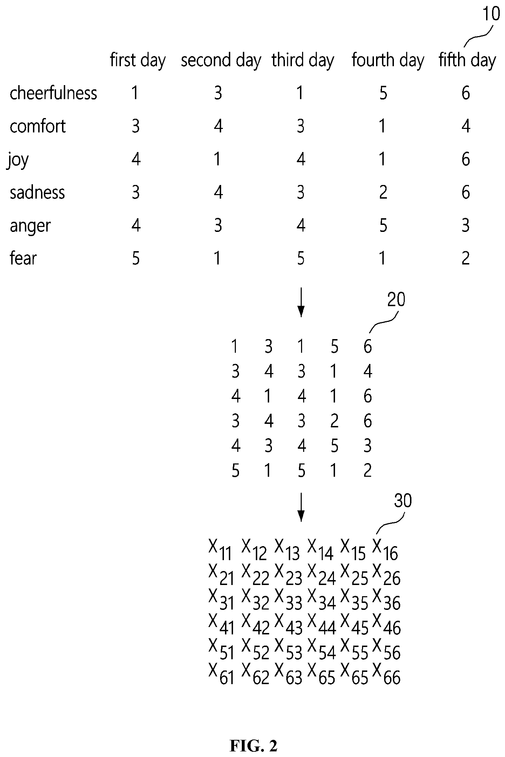

The data generation module 120 may generate emotional network data based on the time-series data provided from the measurement module 110, and may generate interconnection network data representing correlation between respective emotional symptoms. According to an embodiment, the time-series data may be values voluntarily measured by an individual, or may be values estimated based on the usage history of the digital device. For example, the emotional network data may be a matrix in which each row represents time-series data generated for each emotional symptom, and the interconnection network data may be a symmetric matrix in which each component represents a correlation coefficient between the time-series data. Detailed examples of the emotional network data and the interconnection network data will be described in more detail with reference to FIG. 2.

When generating the emotional network data, the data generation module 120 may perform pre-processing, such as normalizing a plurality of time-series data to the same scale, respectively, when scales of the plurality of time-series data composed of emotional symptom measurement values are different from each other, and interpolating a missing value in each of the time-series data. The emotional network data and the interconnection network data, which are generated through the data generation module 120, may be provided to the prediction module 130.

The prediction module 130 may predict a future depression symptom by applying a machine learning algorithm to the emotional network data or the interconnection network data, which are provided from the data generation module 120. To this end, the prediction module 130 may use a machine learning model trained to predict depression symptoms through a large amount of emotional symptom measurement values obtained in a clinical field. For example, the prediction module 130 may predict whether a specific individual's future depression symptoms will worsen by applying a Graph Convolutional Neural Network (Graph CNN) algorithm to the emotional network data and the interconnection network data (i.e., by inputting the network data into a trained Graph CNN), but the present disclosure is not limited thereto. The prediction module 130 may predict depression symptoms by using various other machine learning algorithms other than the Graph CNN.

In detail, the prediction module 130 may predict whether depression symptoms will worsen in the future by applying the machine learning algorithm to the interconnection network data. Since a plurality of emotional symptoms are complexly involved in each symptom of depression, whether the depression symptoms will worsen may be predicted through the interconnection network data including correlation coefficients between the emotional symptoms. Further, the prediction module 130 may predict a change trend of a specific emotional symptom that may affect the worsening of depression symptoms by using the interconnection network data and a measurement value of a specific emotional symptom included in the emotional network data together. In this case, the measurement value of the emotional symptom may function as a feature that may be used in the machine learning algorithm. A prediction result through the prediction module 130 may be displayed to a user as a graphical interface.

Furthermore, the prediction module 130 may provide feedback to the user to change a corresponding emotional symptom in a direction of alleviating depression symptoms based on the predicted result of the specific emotional symptom. For example, when positive emotional symptoms of the user are predicted to weaken, the prediction module 130 may provide feedback to strengthen the positive emotional symptoms of the user. When negative emotional symptoms are predicted to worsen, the prediction module 130 may provide feedback to alleviate the negative emotional symptoms of the user. For example, the feedback may include playing music or videos frequently listened to by the user, recommending content liked by the user, presenting photos liked by the user, and the like, based on the user's usage history of the digital device.

The depression prediction apparatus 100 may be implemented in a computing device equipped with a computing means and a communication means, such as a smartphone, a desktop, a laptop, or the like. For example, the depression prediction apparatus 100 may be implemented as an application on a smartphone to measure emotional symptoms of a smartphone user for a plurality of days, generate emotional network data and interconnection network data based on the emotional symptoms measured for the plurality of days and predict depression symptoms of the smartphone user by applying the machine learning algorithm to the generated network data.

In addition, the depression prediction apparatus 100 may communicate with an external server (e.g., a server in which clinical data related to depression is stored) through all types of wired/wireless networks, such as a local area network (LAN), a wide area network (WAN), a mobile radio communication network, or a wireless broadband internet (WiBro), if necessary.

FIG. 2 exemplarily illustrates emotional network data and interconnection network data according to an embodiment of the present disclosure. Hereinafter, the description will be given with reference to FIG. 1 together with FIG. 2.

It is assumed that the measurement module 110 has measured cheerfulness, comfort, and joy as positive emotional symptoms, and sadness, anger, and fear as negative emotional symptoms for five days (10). In this case, the data generation module 120 may generate emotional network data 20 in a matrix form of 6 rows and 5 columns by using time-series data of each emotional symptom. Each row of the emotional network data 20 represents six different types of emotional symptoms, and each column represents a time point from the 1st day to the 5th day. In addition, the data generation module 120 may generate interconnection network data 30 in a matrix form of 6 rows and 6 columns based on the emotional network data 20. For example, in the interconnection network data 30, x12 may represent a correlation coefficient between time-series data corresponding to cheerfulness and time-series data corresponding to comfort, and x42 may represent a correlation coefficient between time-series data corresponding to sadness and time-series data corresponding to comfort.

Further, although not shown in FIG. 2, as described with reference to FIG. 1, when there is a missing value in the emotional symptom measurement values 10, the missing value may be interpolated by the data generation module 120, and when scales of the measurement values 10 are different from each other, the measurement values 10 may be normalized by the data generation module 120. The emotional network data 20 and the interconnection network data 30 of FIG. 2 may be used for the prediction module 130 to perform a depression symptom prediction operation.

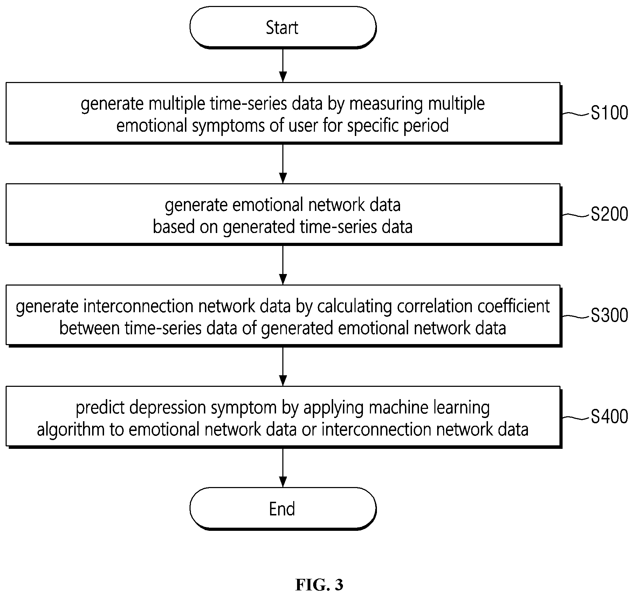

FIG. 3 is a flow chart illustrating a method for predicting depression according to an embodiment of the present disclosure. For reference, FIG. 3 illustrates steps/operations performed in the depression prediction apparatus 100 of FIG. 1. Accordingly, in the following description, when a subject of a specific step/operation is omitted, it may be understood that the corresponding step/operation is performed in the depression prediction apparatus 100 of FIG. 1. Hereinafter, the description will be given with reference to FIG. 1 together with FIG. 3.

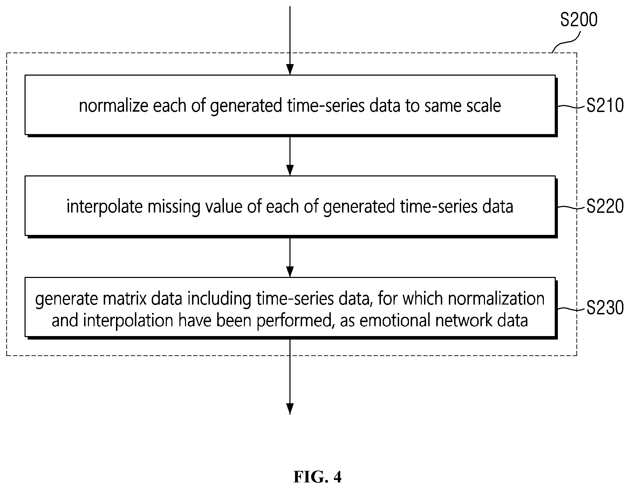

In step S100, the measurement module 110 of the depression prediction apparatus 100 may generate a plurality of time-series data obtained by measuring a plurality of emotional symptoms of a user for a specific period. For example, the measurement module 110 may receive a result of measuring the plurality of emotional symptoms from the user for the specific period. For example, the emotional symptoms may include a plurality of positive emotional symptoms and a plurality of negative emotional symptoms, and the measurement result may be a value quantified on an arbitrary scale (e.g., a Likert scale). In step S200, the data generation module 120 of the depression prediction apparatus 100 may generate emotional network data based on the plurality of time-series data generated in the measurement module 110. Each row of the emotional network data may represent time-series data corresponding to each emotional symptom. Step S200 will be described with reference to FIG. 4.

FIG. 4 is a detailed flow chart illustrating a step S200 of generating emotional network data of FIG. 3. In step S210, the data generation module 120 may normalize each of the generated time-series data to the same scale. For example, the data generation module 120 may use min-max normalization, Z-score normalization or the like as a normalization method, but the present disclosure is not limited thereto. Next, in step S220, the data generation module 120 may interpolate a missing value of each of the generated time-series data. Afterwards, in step S230, the data generation module 120 may generate matrix data including the plurality of time-series data for which the normalization and the interpolation have been performed, as the emotional network data.

Referring back to FIG. 3, in step S300, the data generation module 120 of the depression prediction apparatus 100 may generate interconnection network data by calculating a correlation coefficient between the time-series data of the generated emotional network data. For example, the correlation coefficient may be calculated as a Pearson correlation coefficient.

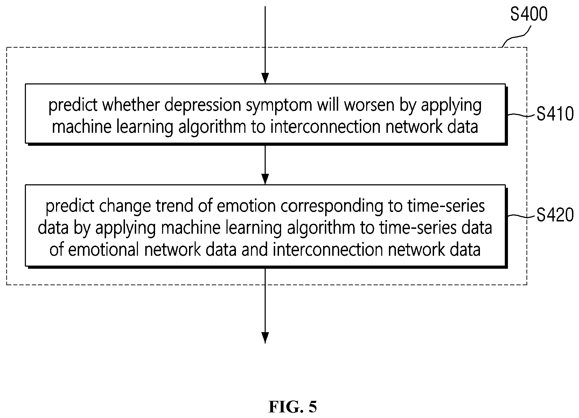

In step S400, the prediction module 130 of the depression prediction apparatus 100 may predict a depression symptom of the user after a specific period by applying a machine learning algorithm to the emotional network data or the interconnection network data. To this end, the prediction module 130 may use a machine learning model trained to predict the depression symptom by receiving the emotional network data or the interconnection network data. For example, the prediction module 130 may use a graph convolutional neural network algorithm as the machine learning algorithm. Step S400 will be described in more detail with reference to FIG. 5.

FIG. 5 is a detailed flow chart illustrating a step S400 of predicting depression symptoms of FIG. 3. In step S410, the prediction module 130 may predict whether the depression symptom will worsen by applying the machine learning algorithm to the interconnection network data. For example, since a plurality of emotional symptoms may complexly affect the depression symptom, the depression symptom may be predicted through correlation of the interconnection network data. Furthermore, in step S420, the prediction module 130 may predict a change trend of an emotion corresponding to the time-series data by applying the machine learning algorithm to the time-series data of the emotional network data and the interconnection network data. In this case, the time-series data is used as a feature related to the machine learning algorithm.

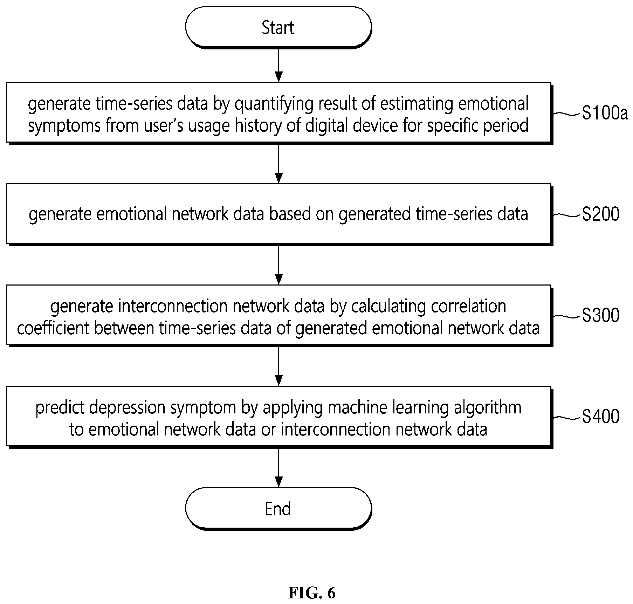

FIG. 6 is a flow chart illustrating a method for predicting depression according to another embodiment of the present disclosure. Hereinafter, the description will be given with reference to FIG. 1 together with FIG. 6, and a redundant description of FIG. 3 will be omitted.

In step S100a, the measurement module 110 of the depression prediction apparatus 100 may generate a plurality of time-series data by quantifying a result of estimating a plurality of emotional symptoms from a user's usage history of a digital device for a specific period. For example, the usage history of the digital device may include the user's search history, content of documents read on the web, content of posts written on the SNS, and content of posts "liked" on the SNS. The estimation of the emotional symptoms may be performed by extracting keywords related to emotions from text acquired from such usage history. The steps S200 to S400 are performed in the same manner as described with reference to FIG. 3.

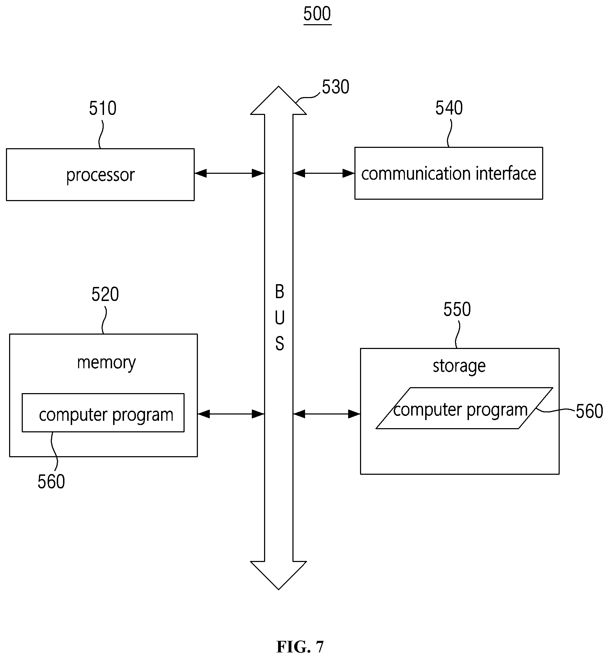

FIG. 7 is a block diagram illustrating hardware configuration of a computing device 500 for predicting depression according to an embodiment of the present disclosure.

Referring to FIG. 7, the computing device 500 may include one or more processors 510, a bus 530, a communication interface 540, a memory 520 into which a computer program executed by the processor 510 is loaded, and a storage 550 for storing the computer program 560. However, only components related to the embodiment of the present disclosure are illustrated in FIG. 7. Accordingly, it will be apparent to those skilled in the art to which the present disclosure pertains that the computing device may further include other general-purpose components in addition to the components shown in FIG. 7. That is, the computing device 500 may further include various components in addition to the components shown in FIG. 7. Also, in some cases, the computing device 500 may be configured in a form in which some of the components shown in FIG. 7 are omitted. Hereinafter, each component of the computing device 500 will be described.

The processor 510 may control the overall operation of each component of the computing device 500. The processor 510 may include at least one of a central processing unit (CPU), a micro processor unit (MPU), a micro controller unit (MCU), a graphic processing unit (GPU), or any type of processor well known in the technical field of the present disclosure. In addition, the processor 510 may perform computation for at least one application or program for executing operations/methods according to the embodiments of the present disclosure. The computing device 500 may include one or more processors.

Next, the memory 520 may store various types of data, commands and/or information. The memory 520 may load a computer program 560 from the storage 550 to execute the operations/methods according to the embodiments of the present disclosure. The memory 520 may be implemented as a volatile memory such as RAM, but the present disclosure is not limited thereto.

Next, the bus 530 may provide a communication function between the components of the computing device 500. The bus 530 may be implemented as various types of buses such as an address bus, a data bus, and a control bus.

Next, the communication interface 540 may support wired/wireless Internet communication of the computing device 500. The communication interface 540 may support various communication modes other than Internet communication. To this end, the communication interface 540 may be configured to include a communication module well known in the technical field of the present disclosure.

The storage 550 may non-temporarily store one or more computer programs 560. The storage 550 may include a nonvolatile memory such as a Read Only Memory (ROM), an Erasable Programmable ROM (EPROM), an Electrically Erasable Programmable ROM (EEPROM) and a flash memory, a hard disk, a detachable disk, or any type of computer-readable recording medium well known in the technical field to which the present disclosure pertains.

Next, the computer program 560 may include one or more instructions to allow the processor 510 to perform the operations/methods according to various embodiments of the present disclosure when loaded into the memory 520. That is, the processor 510 may perform the operations/methods according to various embodiments of the present disclosure by executing the loaded one or more instructions.

For example, the computer program 560 may include instructions for: generating a plurality of time-series data obtained by measuring a plurality of emotional symptoms of a user for a specific period; generating emotional network data based on the generated plurality of time-series data; generating interconnection network data by calculating a correlation coefficient between the time-series data of the generated emotional network data; and predicting a depression symptom by applying a machine learning algorithm to the emotional network data or the interconnection network data. Alternatively, according to an embodiment, the computer program 560 may further include an instruction for generating the plurality of time-series data by quantifying a result of estimating the plurality of emotional symptoms from the user's usage history of a digital device for the specific period.

According to an embodiment of the present disclosure, it is not necessary to visit a medical institution to measure biological biomarkers for diagnosing depression, and demands for personal information are minimal in terms of using digital biomarkers, so that utility and individual acceptability may be significantly improved. In addition, since the method for predicting depression according to an embodiment of the present disclosure shows high prediction accuracy even without large-scale data, whether depression symptoms will worsen and a change trend of a specific emotion may be easily and quickly predicted in advance by using the method.

That is, according to an embodiment of the present disclosure, it is possible to predict the possibility that a potential patient, who is not currently diagnosed with depression, will suffer from depression in the future. In other words, according to an embodiment of the present disclosure, as it becomes easier for depressed patients, who have difficulty visiting a hospital, to voluntarily diagnose whether their symptoms will worsen, it is possible to pre-screen and prevent future potential depressed patients, unlike simply screening and treating current depressed patients.

Various embodiments and the effects thereof according to the present disclosure have been mentioned with reference to FIGS. 1 through 7. The effects according to the technical spirit of the present disclosure are not limited to those mentioned above, and other effects not mentioned will be clearly understood by one of ordinary skill in the art from the description below.

While all components comprising the embodiments of the present disclosure have been described as being combined or operating in conjunction, it should not be understood that the present disclosure is limited to such embodiments. That is, within the scope of the objectives of the present disclosure, all such components can selectively be combined and operate in one or more configurations.

Although operations are illustrated in a specific order in the drawings, it should not be understood that the operations must be performed in that specific order or sequentially, or that all the illustrated operations are required to achieve desired results. In certain circumstances, multitasking and parallel processing may be advantageous. Furthermore, the separation of various components in the described embodiments should not be understood as necessary, and the described program components and systems can generally be integrated into a single software product or packaged into multiple software products.

While the embodiments of the present disclosure have been described with reference to the attached drawings, it will be understood by one skilled in the art that the present disclosure can be implemented in other specific forms without departing from the technical spirit or essential characteristics thereof. Therefore, the described embodiments should be considered in all respects as illustrative and not restrictive. The scope of the present disclosure is to be interpreted by the following claims, and all technical spirits within the equivalent scope are to be interpreted as included within the rights of the present disclosure.

## Claims

1. A method for predicting depression, performed by a computing device, the method comprising:
generating a plurality of time-series data by measuring a plurality of emotional symptoms of a user for a specific period;
generating emotional network data based on the generated time-series data;
generating interconnection network data by calculating a correlation coefficient between the time-series data of the generated emotional network data; and
predicting a depression symptom of the user after the specific period by applying a machine learning algorithm to the emotional network data or the interconnection network data.

2. The method of claim 1, wherein the plurality of emotional symptoms include a plurality of positive emotional symptoms and a plurality of negative emotional symptoms.

3. The method of claim 1, wherein the generating emotional network data includes:
normalizing each of the generated time-series data to a same scale;
interpolating a missing value of each of the generated time-series data; and
generating matrix data including the plurality of time-series data, for which the normalization and the interpolation have been performed, as the emotional network data.

4. The method of claim 1, wherein the predicting a depression symptom includes:
predicting whether the depression symptom will worsen by applying the machine learning algorithm to the interconnection network data; and
predicting a change trend of an emotion corresponding to the time-series data by applying the machine learning algorithm to the time-series data of the emotional network data and the interconnection network data.

5. The method of claim 1, wherein the machine learning algorithm is a Graph Convolutional Neural Network (Graph CNN) algorithm.

6. The method of claim 1, wherein the generating a plurality of time-series data includes generating the plurality of time-series data by receiving a result of measuring the plurality of emotional symptoms of the user from the user for the specific period.

7. The method of claim 1, wherein the generating a plurality of time-series data includes generating the plurality of time-series data by quantifying a result of estimating the plurality of emotional symptoms from the user's usage history of a digital device for the specific period.

8. The method of claim 6, wherein the plurality of emotional symptoms are estimated by extracting keywords representing emotions from text acquired from the usage history of the digital device.

9. A computing device comprising:
a processor; and
a memory storing instructions,
wherein the instructions, when executed by the processor, cause the processor to perform operations of:
generating a plurality of time-series data by measuring a plurality of emotional symptoms of a user for a specific period;
generating emotional network data based on the generated time-series data;
generating interconnection network data by calculating a correlation coefficient between the time-series data of the generated emotional network data; and
predicting a depression symptom of the user after the specific period by applying a machine learning algorithm to the emotional network data or the interconnection network data.

10. The computing device of claim 9, wherein the plurality of emotional symptoms include a plurality of positive emotional symptoms and a plurality of negative emotional symptoms.

11. The computing device of claim 9, wherein the operation of generating emotional network data includes:
normalizing each of the generated time-series data to a same scale;
interpolating a missing value of each of the generated time-series data; and
generating matrix data including the plurality of time-series data, for which the normalization and the interpolation have been performed, as the emotional network data.

12. The computing device of claim 9, wherein the predicting a depression symptom includes:
predicting whether the depression symptom will worsen by applying the machine learning algorithm to the interconnection network data; and
predicting a change trend of an emotion corresponding to the time-series data by applying the machine learning algorithm to the time-series data of the emotional network data and the interconnection network data.

13. The computing device of claim 9, wherein the machine learning algorithm is a Graph Convolutional Neural Network (Graph CNN) algorithm.

14. The computing device of claim 9, wherein the generating a plurality of time-series data includes generating the plurality of time-series data by receiving a result of measuring the plurality of emotional symptoms of the user from the user for the specific period.

15. The computing device of claim 9, wherein the generating a plurality of time-series data includes generating the plurality of time-series data by quantifying a result of estimating the plurality of emotional symptoms from the user's usage history of a digital device for the specific period.

16. The computing device of claim 15, wherein the plurality of emotional symptoms are estimated by extracting keywords representing emotions from text acquired from the usage history of the digital device.

17. An apparatus for predicting depression, comprising:
a measurement module generating a plurality of time-series data by measuring a plurality of emotional symptoms of a user for a specific period;
a data generation module generating emotional network data based on the generated time-series data, and generating interconnection network data by calculating a correlation coefficient between the time-series data of the generated emotional network data; and
a prediction module predicting a depression symptom of the user after the specific period by applying a machine learning algorithm to the emotional network data or the interconnection network data.
